# FASCICULE DE BREVET EUROPEEN

(11) **EP 1 610 819 B1**
(45) Date de publication et mention de la délivrance du brevet: **19.06.2013**
(21) Numéro de dépôt: 04742459.3
(22) Date de dépôt: 08.04.2004
(51) Int. Cl.: A61K 39/395, A61K 47/00, A61K 47/26

(54) **FORMULATION STABILISANTE POUR COMPOSITIONS D'IMMUNOGLOBULINES G SOUS FORME LIQUIDE ET SOUS FORME LYOPHILISEE**
STABILISIERENDE FORMULIERUNG FÜR IMMUNOGLOBULIN G ZUSAMMENSETZUNGEN IN FLÜSSIGER ODER LYOPHILISIERTER FORM
STABILISING FORMULATION FOR IMMUNOGLOBULIN G COMPOSITIONS IN LIQUID FORM AND IN LYOPHILISED FORM

(30) Priorité: 09.04.2003 FR 0304388
(43) Date de publication de la demande: 04.01.2006
(73) Titulaire: Laboratoire Français du Fractionnement et des Biotechnologies, 91940 Les Ulis (FR)
(72) Inventeur: BARDAT, Annie, F-91470 Limours (FR); BEGIN, Edith, F-91940 lES uLIS (FR); KHANDOUDI, Nassirah, F-91940 Les Ulis (FR); JUST, Olivier, F-92260 Fontenay Aux Roses (FR); CHTOUROU, Sami, F-78990 Elancourt (FR); SCHMITTHAEUSLER, Roland, F-78180 Montigny le Bretonneux (FR)
(74) Mandataire: Hirsch & Associés
(86) Numéro de dépôt international: PCT/FR2004/000871
(87) Numéro de publication internationale: WO 2004/091656

(56) Documents cités:
- EP-A- 0 196 761
- WO-A-97/04801
- WO-A-98/44948
- WO-A-03/009817
- US-A- 5 945 098

## Description

L'invention concerne une formulation stabilisante, pharmaceutiquement acceptable, pour la stabilisation et la conservation de compositions d'immunoglobulines G (IgG) soit sous forme liquide, soit sous forme lyophilisée.

De nombreuses pathologies par exemple d'origine autoimmune sont actuellement traitées par des concentrés d'IgG ce qui a engendré une situation de pénurie en Europe et aux Etats Unis d'Amérique au cours de ces dernières années.

Il y a, à cet effet, un besoin grandissant de produire des concentrés d'IgG, habituellement conditionnés à des pH acides et injectables par voie intraveineuse, à partir par exemple de plasmas humains. Avec l'essor de ces besoins en IgG, la stabilisation de ces concentrés d'IgG injectables par voie intraveineuse (IgGIV) en vue de leur utilisation thérapeutique et de leur conservation soit sous forme liquide, soit sous forme lyophilisée, revêt un caractère fondamental.

A cet égard, on sait qu'il est nécessaire de stabiliser les IgGIV pour éviter notamment la formation d'agrégats (oligomères et polymères) susceptibles d'activer le système du complément avec des risques associés de réactions anaphylactiques. Par ailleurs, la présence de dimères dans les IgGIV a été corrélée à des baisses de pression artérielle *in vivo* (Bleeker W.K. et al, Blood, 95, 2000, p. 1856-1861). D'autres dégradations physicochimiques peuvent également intervenir au cours de la conservation des IgG comme, entre autres, l'oxydation et l'hydrolyse.

La stabilisation des formes lyophilisées ou liquides des IgG nécessite donc l'ajout de composés, classiquement choisis parmi les sucres et les acides aminés, afin d'obtenir non seulement des compositions d'IgG non dégradées appropriées à un usage thérapeutique mais également des compositions d'IgG présentant une stabilité accrue au stockage.

La stabilisation des formes lyophilisées de compositions protéiniques et notamment d'IgG, par l'ajout de stabilisants spécifiques, a fait l'objet de très nombreuses études. Celles citées dans les publications scientifiques de M. Pikal, "Freeze-Drying of Proteins, Part 2 : Formulation Selection", Biopharm. 3(9); pp.26-30 (1990) et de Arakawa et al, Pharm. Res., 1991, 8(3), p. 285-291, montrent que l'ajout d'un excipient dans des compositions protéiniques avant lyophilisation, augmente la stabilité au cours de la lyophilisation et/ou la stabilité du produit lyophilisé lors du stockage. Parmi ces stabilisants, certains sont toutefois connus comme étant des agents précipitants de protéines supérieures à environ 100 kDa. Ainsi, l'utilisation de polyéthylèneglycol (PEG) 3000-6000 est rhédibitoire dans la phase de congélation en vue de la lyophilisation de compositions protéiniques correspondantes. Osterberg et al (Pharm. Res., 1997, 14(7), p. 892-898) ont montré l'efficacité d'un mélange d'histidine, de saccharose, d'un tensioactif non ionique et de chlorure de sodium pour la stabilisation des formes lyophilisées du facteur VIII recombinant et l'ajout de PEG n'en a pas amélioré la stabilité. En outre, Guo et al Biomacromol., 2002, 3(4), p. 846-849) précisent que la lyophilisation de la peroxydase de raifort en présence de PEG, ne permet pas d'en conserver la structure native. La présence de PEG ne paraît donc pas souhaitable.

Des compositions d'IgGIV lyophilisées sont disponibles dans le commerce, par exemple sous les noms de marques Polygam^{™} (American Red Cross), Gammar IV^{™} (Armour Pharmaceutical Company) et Venoglobulin^{™}I (Alpha) contenant comme stabilisants du glucose à 2%, du saccharose à 5% et du D-mannitol à 2% respectivement.

Le brevet US 5 945 098 décrit des compositions d'IgG stables au stockage comprenant, à titre de stabilisants, un ou plusieurs acides aminés, tel que la glycine, un détergent non ionique, du polyéthylène glycol (PEG) (nécessairement présent dans toutes ces compositions), et éventuellement d'autres composés, tels que la polyvinylpyrrolidone (PVP), des sucres et des sels.

La demande EP 0 196 761 décrit des composés qui ne peuvent être utilisés que comme stabilisants au cours de la pasteurisation de solutions d'IgG. Parmi ceux-ci, un stabilisant principal est un sucre (monosaccharide, disaccharide ou sucre alcoolique), auquel un stabilisant secondaire est ajouté pouvant représenter au moins un acide aminé neutre, un sel neutre d'acide inorganique, un sel d'acide carboxylique et un tensioactif.

La demande de brevet international WO 97/04801 décrit l'effet de stabilisation de formulations d'anticorps monoclonaux lyophilisés (de type immmunoglobulines G et E) comprenant des excipients spécifiques. Parmi ces excipients, la combinaison glycine/mannitol n'est pas retenue pour défaut d'efficacité au regard d'autres combinaisons telles que saccharose/glycine et saccharose/mannitol.

On constate, toutefois, que des stabilisants appropriés aux formes lyophilisées des IgGIV peuvent être totalement inefficaces pour des compositions d'IgGIV liquides.

Ainsi, les compositions d'IgGIV liquides, disponibles dans le commerce, comprennent des stabilisants spécifiques différents de ceux utilisés pour la forme lyophilisée correspondante. A titre d'exemple, les compositions liquides d'IgGIV contenant comme stabilisants du maltose à 10%, de la glycine de 0,16 à 0,24 M et du D-sorbitol à 5% sont respectivement connues sous les noms de marque Gamimune N^{™}, Gamimune N^{™} 10% (Miles Inc.) et Venoglobulin^{™} (Alpha).

La nature différente des composés utilisés pour la stabilisation des compositions d'IgG sous forme liquide et sous forme lyophilisée, a amené certains auteurs à rechercher des stabilisants ou mélanges de stabilisants identiques permettant de conserver les compositions d'IgG sous les deux formes à la fois. A cet égard, des études récentes ont porté sur la stabilisation de compositions d'IgGIV, Vigam-S et Vigam Liquid (noms de marques de National Blood Authority, Angleterre) liquides et après lyophilisation (Vigam-S) comprenant un mélange identique de stabilisants à savoir l'albumine et le saccharose (K. Chidwick et al, Vox Sanguinis, 77, 204-209, 1999). Toutefois, la solution Vigam Liquid est conditionnée à un pH acide (pH 5) ce qui présente l'inconvénient de transformer, par hydrolyse, le saccharose en sucres réducteurs (fructose et glucose) qui se condensent avec les résidus aminés de la lysine des IgG et de l'albumine pour donner une base de Schiff instable évoluant en des produits de Maillard (brunissement de la solution). Il n'est bien entendu pas satisfaisant d'utiliser des excipients qui évoluent au cours de la conservation des IgG car la maîtrise de la réaction n'est pas possible, une fois celle-ci initiée.

Par ailleurs, certains des stabilisants cités précédemment, comme le maltose ou le saccharose, ne peuvent être utilisés sans risques chez des sujets présentant des insuffisances rénales et/ou souffrant de diabète.

Afin de pallier les inconvénients précédents, la Demanderesse s'est attachée à la mise au point d'une formulation stabilisante unique, pharmaceutiquement acceptable, répondant à l'objectif d'assurer la stabilisation des deux formes de conservation envisagées des IgG à la fois et de conserver, voire améliorer, l'efficacité thérapeutique de ces IgG.

Une telle formulation stabilisante présente notamment l'avantage de ne nécessiter la mise en oeuvre que d'une seule formulation ce qui facilite le contrôle des matières premières et entraîne une réduction des coûts de fabrication associés à des simplifications des schémas de production.

A cet effet, partant du constat que des sucres et des acides aminés sont utilisés comme stabilisants, la Demanderesse a montré, sur la base d'essais préliminaires, que certains d'entre eux apportaient à des compositions d'IgG liquides des propriétés protectrices contre les dénaturations thermique et par agitation provoquées mais avec des résultats variables quant à la nature du sucre et de l'acide aminé choisis et que, en outre, les effets de stabilisation d'un mélange, par exemple de deux constituants, ne pouvaient être déduits de l'effet de stabilisation obtenu par chaque constituant pris individuellement. De plus, parmi les sucres essayés, certains d'entre eux n'étaient pas stables à des pH acides correspondant au milieu de conditionnement optimal des compositions d'IgG liquides.

Par ailleurs, les stabilisants retenus après les essais préliminaires ne permettaient pas de minimiser l'instabilité des compositions d'IgG liquides face à l'oxydation provoquée. La Demanderesse a alors ajouté un détergent non ionique tel que le Tween^{®}80 ou le Triton^{®} X 100 et obtenu des résultats satisfaisants.

L'invention concerne une formulation stabilisante pour une composition d'immunoglobulines G polyclonales, caractérisée en ce qu'elle est constituée par un sucre alcoolique, la glycine en une concentration comprise entre 7 g/l et 10 g/l et un détergent non ionique en une concentration comprise entre 20 et 50 ppm, pour être ainsi appropriée à la stabilisation de compositions d'immunoglobulines G polyclonales sous forme liquide et à la stabilisation de compositions d'immunoglobulines G polyclonales sous forme lyophilisée.

La formulation selon l'invention est constituée desdits sucre alcoolique, glycine et détergent non ionique. Une telle formulation stabilisante exclusivement constituée de ces trois composés selon l'invention, présente l'avantage d'offrir une stabilisation conjointe des compositions d'IgG lyophilisées et non lyophilisées, et une réduction des durées et des coûts de préparation à l'échelle industrielle grâce à la présence d'un nombre minimal efficace de stabilisants.

Dans le cadre de l'invention, les compositions d'IgG liquides signifient aussi bien des solutions aqueuses de concentrés d'IgG polyclonales, directement obtenues par fractionnement de plasma humain, que celles reconstituées dans un milieu aqueux approprié après lyophilisation des précédentes. Le milieu aqueux de reconstitution représente de l'eau pour préparation injectable (eau PPI) pouvant contenir des excipients pharmaceutiquement acceptables et compatibles avec les IgG. Ces compositions d'IgG peuvent ensuite subir des étapes spécifiques d'inactivation/élimination de virus. De préférence, les méthodes de fractionnement du plasma représentent celles décrites par Cohn et al (J. Am. Chem. Soc., 68, 459, 1946), Kistler et al. (Vox Sang., 7, 1962, 414-424), Steinbuch et al (Rev. Franç. Et. Clin. et Biol., XIV, 1054, 1969) et dans la demande de brevet WO 94/9334.

Parmi les sucres envisagés, les sucres alcooliques ont été choisis par la Demanderesse sur des critères de stabilité à des pH acides de conditionnement des compositions d'IgG, ce qui évite des réactions de Maillard sur les immunoglobulines G, de compatibilité pharmaceutique et sur des critères relatifs à leur action stabilisante uniquement de compositions liquides d'IgG ou bien de celles lyophilisées. En effet, on a observé qu'un sucre alcoolique donné utilisé comme seul stabilisant ne pouvait correspondre qu'à une forme liquide.

Parmi les sucres alcooliques, ceux utilisés de préférence selon l'invention représentent le mannitol, le sorbitol ou leurs isomères et, de façon la plus préférée, le mannitol.

La glycine, présente dans la formulation stabilisante de l'invention, est connue pour être appropriée à la stabilisation de compositions d'IgG mais sous forme liquide uniquement.

L'addition d'un détergent non ionique a, par synergie, amélioré, de façon surprenante, l'effet protecteur de la formulation. Les détergents non ioniques appropriés sont avantageusement choisis dans le groupe constitué par le Tween^{®}80 (polyoxyéthylènesorbitanne-monooléate), le Tween^{®}20 (polyoxyéthylènesorbitanne-monolaurate), le Triton^{®} X 100 (octoxinol 10) et le Pluronic^{®}F68 (polyéthylènepolypropylène glycols). De préférence, le Tween^{®}80 et le Triton^{®} X100 ont été utilisés.

Les concentrations de ces composés seront choisies par l'homme du métier de façon à obtenir l'effet de stabilisation voulu des compositions d'IgG lyophilisées et non lyophilisées.

De préférence, les concentrations en mannitol sont comprises entre 30 g/l et 50 g/l.

L'invention concerne également les compositions d'IgG sous forme liquide et/ou sous forme lyophilisée comprenant la formulation stabilisante de l'invention, pouvant en outre être à usage thérapeutique et notamment injectables par voie intraveineuse. Ces compositions d'IgG, sous forme liquide et/ou sous forme lyophilisée, grâce à la présence de la formulation stabilisante de l'invention, présentent un taux de dimères inférieur à 7%, après un stockage durant une période de 24 mois à 4°C. On observe que le stockage des compositions d'IgG sous forme liquide pendant 6 mois à température ambiante, engendre un taux de polymères bien en-deça des normes fixées par la Pharmacopée Européenne (3%), soit inférieur à environ 0,3%. Les compositions d'IgG sous forme lyophilisée présentent un taux de polymère environ 10 fois inférieur à celui toléré, après leur stockage pendant 12 mois à température ambiante ou pendant 6 mois à 40°C.

L'invention concerne en outre l'utilisation d'une formulation stabilisante selon l'invention, pour la stabilisation de compositions d'immunoglobulines G sous forme liquide directement obtenues par fractionnement de plasma humain, sous forme lyophilisée et de celles obtenues après reconstitution dans un milieu aqueux approprié des formes lyophilisées.

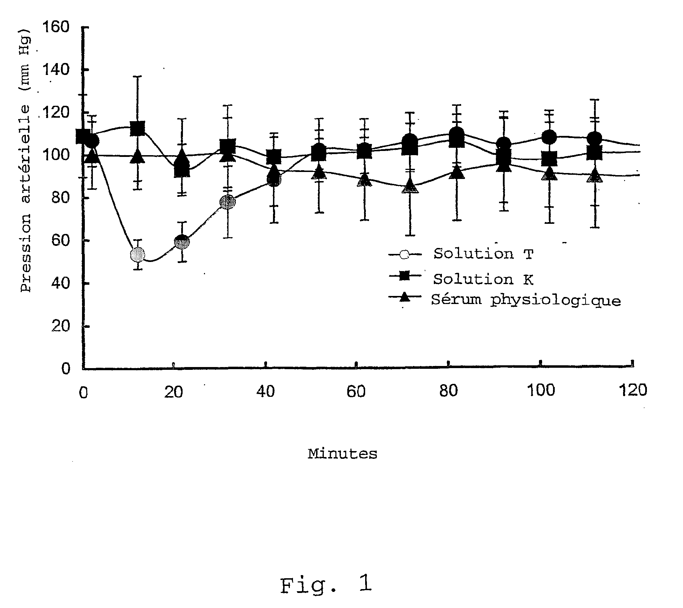

Les exemples suivants illustrent l'invention sans toutefois en limiter la portée, en référence à la figure 1 qui représente un tracé des variations de la pression artérielle chez des rats en fonction du temps, après injection de différentes compositions d'immunoglobulines G ci-dessus.

### Exemple 1 : Elaboration de la formulation stabilisante.

On utilise comme composition d'IgG, un concentré obtenu selon la méthode développée par la Demanderesse dans la demande de brevet internationale WO 02/092632. Ce concentré, contenant environ 50 g/l d'IgG, est ajusté à un pH compris entre 4,6 et 4,8 et est soumis à un traitement thermique de 2 heures à 56°C pour éliminer des impuretés thermolabiles.

A ce concentré d'IgG, on ajoute le mannitol, la glycine et le Tween^{®}80 ou le Triton^{®} X100, seuls ou en mélange, (solutions test) dans les concentrations précisées au Tableau 1.

**Tableau 1 : Caractéristiques des solutions test**

| solution test | Mannitol (50 g/l) | Glycine (10 g/l) | Détergent (50 ppm) |
|---|---|---|---|
| A (Témoin) | 0 | 0 | 0 |
| B | 1 | 0 | 0 |
| C | 0 | 1 | 0 |
| D | 1 | 1 | 0 |
| E | 0 | 0 | 1 (Triton^{®} X100) |
| F | 0 | 0 | 1 (Tween^{®}80) |
| G | 1 | 0 | 1 (Triton^{®} X100) |
| H | 1 | 0 | 1 (Tween^{®}80) |
| I | 1 | 1 | 1 (Triton^{®} X100) |
| J | 1 | 1 | 1 (Tween^{®}80) |

| | | | |
|---|---|---|---|
| 0 : Absence du composé considéré 1 : Présence du composé considéré | | | |

Les solutions test sont ensuite soumises à différents essais de stress thermique, d'agitation et d'oxydation afin de déterminer leur degré de dénaturation par l'observation de la présence éventuelle de résidus (particules, agrégats).

Le stress thermique est effectué selon la publication de P. Fernandes et al, Vox Sanguinis, 1980,39, p. 101-112. En résumé, des échantillons de 5 ml de solution test sont placés dans des flacons en verre serti de 10 ml et sont ensuite chauffés au bain-marie à 57°C pendant 4 heures. L'influence du chauffage sur les solutions test est déterminée par mesure de la différence de turbidité après et avant le stress thermique. Plus les valeurs de turbidité mesurées sont faibles plus les solutions d'IgG sont stables face au stress thermique appliqué.

Les essais de stress d'agitation sont effectués comme décrit dans la publication de H. Levine et al, Journal of Parental Science & technology, 1991, vol. 45, n°3, p. 160-165. Ainsi, on place des échantillons de 5 ml de solution test dans des tubes en verre serti de 10 ml protégés de la lumière, puis chaque tube est mis en position couchée sur un agitateur IKA Vibrax VXR (provenant de chez Fisher Scientific, France), et est ensuite agité à 150 tours par minute pendant 18 heures à température ambiante. Les résultats du stress d'agitation sont déterminés par comparaison des aspects visuels des solutions test avant et après le stress appliqué. A cet effet, on définit une échelle de valeurs arbitraires suivantes :
0,25 : solution limpide avec une ou deux particules en suspension ;
0,50 : solution limpide avec quelques fines particules en suspension ;
0,75 : solution limpide avec un peu plus de particules en suspension que pour celle à 0,50 ;
2,0 : aspect visuel légèrement modifié avec plus de particules en suspension ;
5,0 : nombreux filaments ou particules en suspension ;
10,0 : particules plus grosses et agrégats, voire coagulats.

Le stress d'oxydation est réalisé sur des échantillons de 5 ml de solution test placés dans des flacons en verre de 10 ml. La surface du liquide est mise en présence d'une atmosphère riche en oxygène (O₂ > 21%) pendant 3 à 4 s. Après bouchage et agitation des flacons, ceux-ci sont refroidis à une température de 5°C pendant 15 minutes. Les résultats sont déterminés par comparaison des aspects visuels des solutions test avant et après le stress d'oxydation appliqué. Les résultats peuvent être chiffrés selon une échelle de valeurs identique à celle définie pour le stress d'agitation.

Les différents résultats de mesure obtenus après l'application des différents stress précédents, sont présentés dans le Tableau 2.

**Tableau 2**

| Solution test | Turbidit é (NTU*) | Avant stress | Après stress d'agitation | Après stress d'oxydation |
|---|---|---|---|---|
| A | 0,73 | 0,5 | 5,0 | 2,0 |
| B | 0,21 | 0,5 | 3,5 | 2,0 |
| C | 0,63 | 0,5 | 0,5 | 3,5 |
| D | 0,42 | 0,25 | 10,0 | 2,0 |
| E | 0,84 | 0,5 | 1,25 | 1,25 |
| F | 0,36 | 0,5 | 1,25 | 2,0 |
| G | 0,45 | 0,25 | 0,75 | 0,5 |
| H | 0,16 | 0,25 | 0,5 | 1,25 |
| I | 0,41 | 0,25 | 0,5 | 0,75 |
| J | 0,39 | 0,25 | 0,5 | 1,25 |

| | | | | |
|---|---|---|---|---|
| *NTU : Normalized Turbidity Units | | | | |

Les résultats obtenus montrent tout d'abord que l'ajout d'un seul stabilisant à une solution de concentré d'IgG (solutions B, C, E, F), par rapport à la solution témoin A ne comportant pas de stabilisant, permet d'améliorer la protection contre deux des trois stress appliqués. Par ailleurs, la présence conjointe de mannitol et de glycine (solution D) n'est pas souhaitable, les résultats du stress d'agitation étant nettement inférieurs à ceux obtenus avec le mannitol et la glycine seuls (solutions B ou C) ou même par rapport à la solution témoin A. Ce résultat indique l'importance du choix des constituants pour l'élaboration de la formulation stabilisante selon l'invention qui ne peut, par conséquent, être déduit seulement des effets des stabilisants pris individuellement. En revanche, les essais menés sur les solutions test montrent que les formulations spécifiques des stabilisants considérés I et J offrent une protection très satisfaisante contre la dénaturation liée aux trois stress appliqués par rapport à la solution témoin A ne comportant pas de stabilisant. Les solutions test G et H contenant les formulations stabilisantes qui ne sont pas selon l'invention, donnent toutefois également des résultats satisfaisants à ce stade.

### Exemple 2

Afin de déterminer d'une façon quantitative le taux de polymères et notamment de dimères présents dans les solutions test G, H, I et J ayant subi le stress d'agitation selon l'Exemple 1, celles-ci sont soumises à une chromatographie d'exclusion stérique selon le mode opératoire décrit dans la Méthode de la Pharmacopée Européenne (Pharmacopée Européenne, quatrième édition, chap. "Immunoglobuline humaine normale pour administration par voie intraveineuse", Méthode 2.2.29).
Le tableau 3 présente les pourcentages de dimères et polymères obtenus.

**Tableau 3**

| Solution test | Dimères (%) | Polymères (%) |
|---|---|---|
| G | 5,90 | 3,30 |
| H | 7,11 | 3,37 |
| I | 4,98 | 2,17 |
| J | 3,50 | 1,80 |

Les pourcentages les plus faibles de polymères sont obtenus avec les solutions test I et J. Ces résultats confirment que les formulations spécifiques des stabilisants considérés I et J offrent une protection très satisfaisante contre la dénaturation liée au stress d'agitation appliqué, tel que décrit dans l'Exemple 1.

Après sélection des formules I et J, seule la solution test J est retenue pour l'exemple qui suit, car elle contient un détergent non ionique pharmaceutiquement acceptable, à savoir le Tween^{®}80.

### Exemple 3

La solution J sous forme liquide (dénommée ci-après "IgG liquides") est soumise à des essais de stabilité en fonction de la durée de stockage dans des conditions usuelles de température (4°C). On procède à des essais identiques pour la solution J lyophilisée (durée de lyophilisation de 45 ± 3 h), dénommée ci-après "IgG lyophilisées". Ces essais de stabilité sont effectués sur les solutions J liquides, reconstituées le cas échéant avec de l'eau pour préparation injectable. Ils consistent en le suivi sur une période de temps de 24 mois, de l'évolution des quatre paramètres définis ci-après, dont trois sont déterminés en référence aux méthodes indiquées dans la Pharmacopée Européenne, quatrième édition, chap. "Immunoglobuline humaine normale pour administration par voie intraveineuse":
(a) Evolution du taux de dimères déterminé par chromatographie d'exclusion stérique (Méthode 2.2.29),
(b) Evolution de l'activité anticomplémentaire (Méthode 2.6.17), et
(c) Evolution du titre d'anticorps spécifiques contre le virus de l'hépatite B, anti HBs (Méthode 2.7.1).
Le quatrième paramètre (d) définit l'évolution des taux d'IgG3 par un dosage néphélémétrique connu par l'homme du métier, en présence d'anti IgG3 spécifiques (DADE-Behring : kit anti IgG3).

Les mesures sont effectuées respectivement après une période de stockage de 12 mois et de 24 mois suivant la préparation de la solution J liquide (t₀).

Les résultats de mesures obtenus pour chaque essai sont présentés dans les Tableaux qui suivent et les valeurs données représentent les valeurs moyennes de trois essais.

### (a) Taux de dimères : Tableau 4

| | t₀ (%) | t₁₂ mois (%) | t₂₄ mois (%) |
|---|---|---|---|
| IgG liquides | 3,2 ± 0 | 5,0 ± 1,0 | 5,5 ± 1,1 |
| IgG lyophilisées | 3,2 ± 0 | 3,5 ± 0,7 | 5,0 ± 1,0 |

L'augmentation du taux de dimères au cours du temps de stockage reste dans les limites de la précision des essais et n'est pas suffisamment significative pour que l'on puisse constater une dégradation quantitative des compositions considérées.

### (b) Activité anticomplémentaire Tableau 5

| | t₀ (%) | t₁₂ mois (%) | t₂₄ mois (%) |
|---|---|---|---|
| IgG liquides | 35 ± 0 | 30 ± 6 | 28 ± 5 |
| IgG lyophilisées | 31 ± 0 | 31 ± 4 | 31 ± 7 |

On observe une légère baisse de l'activité anticomplémentaire pour les compositions d'IgG liquides tandis celle relative aux IgG lyophilisées n'évolue pas. Cette diminution n'a pas de signification clinique, seule une augmentation de cette activité serait défavorable en termes d'utilisation.

### (c) Dosage anti HBs : Tableau 6

| | t₀ (UI/ml) | t₁₂ mois (UI/ml) | t₂₄ mois (UI/ml) |
|---|---|---|---|
| IgG liquides | 12,0 ± 0 | 11,5 ± 2,5 | 11,0 ± 2,0 |
| IgG lyophilisées | 10,0 ± 0 | 10,0 ± 2,2 | 10,5 ± 2,4 |

Bien qu'une très légère baisse semble se produire dans la composition d'IgG liquides, on n'observe aucune variation importante notamment pour les IgG lyophilisées.

### (d) Taux d'IgG3 Tableau 7

| | t₀ (g/l) | t₁₂ mois (g/l) | t₂₄ mois (g/l) |
|---|---|---|---|
| IgG liquides | 1,1 | 1,0 ± 0,2 | 1,1 ± 0,1 |
| IgG lyophilisées | 1,1 | 0,9 ± 0,1 | 1,1 ± 0,1 |

Les variations observées s'inscrivent dans la marge d'incertitude des valeurs des taux d'IgG3. Elles ne sont donc pas significatives.

Les quatre paramètres analysés ci-dessus démontrent que la formulation de la présente invention est particulièrement appropriée à la stabilisation de compositions d'IgG soit sous forme liquide, soit sous forme lyophilisée pendant 24 mois à une température de 4°C sans évolution notable de celles-ci, qui traduirait, dans le cas contraire, une dégradation du produit et ainsi ne permettrait pas leur utilisation clinique.

### Exemple 4

On utilise comme composition d'IgG, celle définie à l'exemple 1. A ce concentré d'IgG, on ajoute du mannitol, de la glycine et du Tween^{®}80 selon des concentrations respectives de 32 g/l, 7 g/l et 50 ppm. La solution K sous forme liquide ainsi obtenue (dénommée ci-après "IgG liquides") est soumise à des essais de stabilité en fonction de la durée de stockage à 4°C, à température ambiante et à 40°C. On procède à des essais identiques pour la solution K lyophilisée (durée de lyophilisation de 45 ± 3h), dénommée ci-après "IgG lyophilisées". Ces essais de stabilité sont effectués sur les solutions K liquides, reconstituées le cas échéant avec de l'eau pour préparation injectable. Ils consistent en le suivi sur une période de temps de 12 mois, de l'évolution du taux de polymères déterminé par chromatographie d'exclusion stérique en référence à la méthode 2.2.29 indiquée dans la Pharmacopée Européenne, précisée à l'exemple 3. Selon les normes fixées par la Pharmacopée Européenne, le seuil maximal toléré est de 3%.

Les mesures sont effectuées respectivement après une période de stockage de 3, 6 et 12 mois suivant la préparation de la solution K liquide (t₀).

Les résultats de mesures obtenus pour chaque essai, aux trois températures de stockage ci-dessus, sont respectivement présentés dans les Tableaux 8, 9 et 10 qui suivent, et les valeurs données représentent les valeurs moyennes de trois essais.

**Tableau 8**

| T : 4°C | t₀ (%) | t₃ mois (%) | t₆ mois (%) | t₁₂ mois (%) |
|---|---|---|---|---|
| IgG liquides | 0,057±0,00 3 | 0,02±0,005 | 0,2 ±0,03 | - |
| IgG lyophilisée s | 0,23±0,05 | 0,2±0,08 | 0,23±0,09 | 0,18±0,07 |

**Tableau 9**

| T : ambiante | t₀ (%) | t₃ mois (%) | t₆ mois (%) | t₁₂ mois (%) |
|---|---|---|---|---|
| IgG liquides | 0,057±0,00 3 | 0,02±0,01 | 0,19 ±0,03 | - |
| IgG lyophilisée s | 0,23±0,05 | 0,24±0,08 | 0,20±0,1 | 0,20±0,01 |

**Tableau 10**

| T : 40°C | t₀ (%) | t₃ mois (%) | t₆ mois (%) | t₁₂ mois (%) |
|---|---|---|---|---|
| IgG liquides | 0,057±0,00 3 | 1,20±0,08 | 6,23 ±0,03 | - |
| IgG lyophilisée s | 0,47±0,3 | 0,55±0,1 | 0,20±0,1 | - |

La durée de stockage des IgG liquides est de 3 mois à 40°C. Au terme des 6 mois de stockage ce celles-ci à 4°C et à température ambiante, le taux de polymères observé est bien en-deça des normes fixées par la Pharmacopée Européenne. Pour les IgG lyophilisée, le stockage pendant 12 mois à 4°C et à température ambiante engendre un taux de polymère 10 fois inférieur à celui toléré. On observe le même taux après un stockage de 6 mois à 40°C.

### Exemple 5

Cet exemple est destiné à confirmer que les solutions de l'invention, présentant un taux de dimères inférieur à 7%, n'induisent pas d'effets hypotenseurs après leur injection *in vivo*. Selon Bleeker W.K. et al (Blood, 95, 2000, p. 1856-1861), les dimères d'immunoglobuline G ont des propriétés hypotensives *in vivo* d'autant plus importantes que leur teneur dans l'échantillon d'IgG à injecter est élevée. Le traitement de maladies auto-immunes nécessitant l'injection de doses massives d'IgG, peut donc présenter des risques chez des patients souffrant d'hypotension si la teneur des dimères d'IgG n'est pas contrôlée.

L'objectif des études présentées dans cet exemple est d'évaluer et de comparer les effets hémodynamiques de deux solutions d'IgG sous forme liquides, représentant respectivement une solution d'IgG classique (Solution T - IgG : 50 g/l ; saccharose : 100 g/l ; NaCl : 3 g/l, pH : 6,5) présentant un taux de dimères de 11,50% et là solution K de l'exemple précédent présentant un taux de dimères de 6,3%, sur des rats anesthésiés préparés.

### Mode opératoire

Des rats mâles adultes Sprague-Dawley (IFFA-Credo : France) de 180-200 g sont anesthésiés par une injection de intrapéritonéale de pentobarbital (Sanofi - France) à raison de 60 mg/kg. Le rat anesthésié est allongé sur le dos sur un matelas thermostaté à 37°C. Un cathéter placé dans la carotide, relié à un capteur de pression et à un enregistreur classiques, permet de mesurer en continu la pression artérielle. Une canule trachéale permet de dégager les voies respiratoires. Les injections intraveineuses d'IgG (solutions K et T) sont effectuées via un cathéter placé dans la veine jugulaire de l'animal, à raison de 2,66 ml/120 min.

On mesure les variations de la pression artérielle (mm Hg) de trois groupes de 6 rats chacun, en fonction du temps:
- un groupe contrôle, recevant du sérum physiologique,
- un groupe traité, recevant la solution T à raison de 0,65 g/kg, et
- un groupe traité, recevant la solution K à raison de 0,65 g/kg.

Les expériences débutent par une phase préalable de stabilisation de 20 min. L'enregistrement en continu de la pression artérielle débute à t₀-10 min. (t₀ = injection).
Les résultats de ces études sont déduits du tracé de la Figure 1, dans laquelle chaque point du tracé représente une valeur moyenne, avec l'écart type, de 6 expériences. L'analyse statistique est réalisée par une analyse de la variance suivie d'un test de Scheffé connu de l'homme du métier.

Ces résultats montrent que les valeurs de la pression artérielle, préalablement à l'injection des IgG, sont stables et comparables dans les trois groupes de rats. A t₀+10 min, on observe une chute de la pression artérielle qui atteint son minimum à environ t₀+15 min à une valeur inférieure de 50% à la valeur initiale avant injection (passant d'environ 100 mm Hg à environ 50 mm Hg), suivie d'un retour progressif vers des valeurs initiales de la pression atteintes 50 à 60 minutes après le début de l'injection.

### Exemple 6

Cet exemple est destiné à vérifier que la solution J sous forme liquide contribue à la diminution de la viscosité sanguine, par rapport à des solutions comprenant des protéines et des excipients différents. A ce titre, deux essais de sédimentation de globules rouges par pesanteur ont été effectués selon des méthodes connues de l'homme du métier, à température ambiante et à T = 37°C.

### Mode opératoire

Des globules rouges humains de groupe O+ en présence d'une solution anticoagulante de citrate trisodique 0,2 M (1/9, v/v), sont lavés trois fois par une solution saline de PBS de concentration habituelle, pH 7,4. On prépare des solutions aqueuses de protéines dans des excipients différents, dont la solution J qui est ajustée en NaCl 0,15 M et à pH 7. Les protéines et les excipients, ainsi que leur concentrations respectives, sont indiqués au Tableau 11. On prélève 4,5 ml de chacune des solutions protéiniques que l'on place dans un tube gradué en verre de 10 ml. A chaque solution, on ajoute ensuite 0,5 ml de globules rouges lavés. Les mélanges ainsi obtenus sont homogénéisés par 3-4 retournements des tubes bouchés. A la fin de l'homogénéisation, les tubes sont mis au repos sur un présentoir et on mesure le temps nécessaire à l'apparition d'une ligne de décantation nette des globules rouges tangente au ménisque limpide, traduisant la vitesse de sédimentation. Les résultats des essais à la température ambiante figurent au Tableau 11.

**Tableau 11 : Essais à T ambiante**

| Protéine(s) | Solution | Temps de sédimentation au ménisque (min.) | Remarque |
|---|---|---|---|
| - | PBS | 22,70 | Témoin (sans protéine) |
| Albumine (5%) | NaCl : 2 g/l | 35,90 | Protéine oncotique |
| Plasma humain | NaCl : 2 g/l | | Toutes les protéines présentes |
| | Citrate trisodique: 0,02 M | 28,42 | |
| IgG (50 g/l) | Saccharose : 100 g/l | 31,25 | Formulation de l'art antérieur |
| | NaCl : 3 g/l | | |
| Solution J | + NaCl : 0,15 M | 9,25 | Formulation de l'invention |
| Fibrinogène (15 g/l) | Arginine : 40 g/l | | |
| | Citrate trisodique : 2,5 g/l | 12,60 | Présence d'agglutinats |
| | Lysine : 2 g/l | | |

Les résultats obtenus montrent que les globules rouges considérés, en présence d'IgG de la solution J de l'invention, sédimentent le plus rapidement, ce qui est conforme à une diminution de la viscosité du mélange étudié, permettant ainsi une meilleure fluidité du sang.

D'autres essais sont menés en considérant les mélanges ci-dessus (4,5 ml de solutions protéiniques et 0,5 ml de globules rouges lavés) placés dans des tubes gradués en verre de 10 ml et homogénéisés par 3-4 retournements des tubes. Une fois cette opération achevée, ceux-ci sont placés de façon que leur contenu sédimente pendant un laps de temps de 1 heure selon un angle de 45°, à une température de 37°C et on mesure le volume de surnageant pour les mélanges considérés. Les résultats des essais à T = 37°C figurent au Tableau 12.

**Tableau 12 : Essais à T = 37°C**

| Protéine(s) | Solution | Volume de surnageant après 1 heure (ml) |
|---|---|---|
| - | PBS | 2,5 |
| Albumine (5%) | NaCl : 2 g/l | 2,5 |
| Plasma humain | NaCl : 2 g/l | |
| | Citrate trisodique : 0,02 M | 4 |
| IgG (50 g/l) | Saccharose : 100 g/l | 1* |
| | NaCl : 3 g/l | |
| Solution J | + NaCl : 0,15 M | 2,5 |

| | | |
|---|---|---|
| * : surnageant présentant des signes d'hémolyse | | |

L'analyse des résultats du Tableau 12 montre que :
- le volume de surnageant pour le mélange de plasma étant le plus important des mélanges étudiés, celui-ci présente donc la viscosité correspondante la plus faible ;
   les mélanges d'albumine et de solution J de l'invention présentent une vitesse de sédimentation identique (même volume de surnageant) ;
- le mélange d'IgG de l'art antérieur, c'est-à-dire celui comprenant du saccharose et du NaCl, conduit au volume de surnageant le moins important, ce qui signifie que les globules rouges sédimentent plus lentement que dans le mélange J de l'invention, ainsi que dans les autres mélanges étudiés ci-dessus, le surnageant étant en outre de teinte rosée du fait de son hémolyse discrète.

## Revendications

1. Formulation stabilisante pour une composition d'immunoglobulines G polyclonales, **caractérisée en ce qu'**elle est constituée par un sucre alcoolique, la glycine en une concentration comprise entre 7 g/l et 10 g/l et un détergent non ionique en une concentration comprise entre 20 et 50 ppm, pour être ainsi appropriée à la stabilisation de compositions d'immunoglobulines G polyclonales sous forme liquide et à la stabilisation de compositions d'immunoglobulines G polyclonales sous forme lyophilisée.

2. Formulation selon la revendication 1, **caractérisées en ce que** le sucre alcoolique est le mannitol.

3. Formulation selon la revendication 2, **caractérisée en ce que** la concentration de mannitol est comprise entre 30 g/l et 50 g/l.

4. Composition d'immunoglobulines G polyclonales sous forme liquide comprenant, comme stabilisant, la formulation stabilisante selon l'une quelconque des revendications 1 à 3.

5. Composition d'immunoglobulines G polyclonales sous forme lyophilisée, obtenue par lyophilisation d'une composition d'immunoglobulines G polyclonales et de la formulation stabilisante selon l'une quelconque des revendications 1 à 3.

6. Composition d'immunoglobulines G polyclonales selon la revendication 4, **caractérisée en ce qu'**elle présente un taux de polymères inférieur à 0,3% après un stockage de 6 mois à température ambiante.

7. Composition d'immunoglobulines G polyclonales selon la revendication 5, **caractérisée en ce qu'**elle présente un taux de polymères inférieur à 0,3% après un stockage de 12 mois à température ambiante ou de 6 mois à 40°C.

8. Composition d'immunoglobulines G polyclonales selon l'une quelconque des revendications 4 à 7, **caractérisée en ce qu'**elle présente un taux de dimères inférieur à 7% après un stockage durant une période de 24 mois à 4°C.

9. Utilisation d'une formulation stabilisante selon l'une quelconque des revendications 1 à 3, comme stabilisant de compositions d'immunoglobulines G polyclonales sous forme liquide directement obtenues par fractionnement de plasma humain.

10. Utilisation d'une formulation stabilisante selon l'une quelconque des revendications 1 à 3, pour la stabilisation de compositions d'immunoglobulines G polyclonales sous forme lyophilisée.

11. Utilisation d'une formulation stabilisante selon l'une quelconque des revendications 1 à 3, pour la stabilisation de compositions d'immunoglobulines G polyclonales sous forme liquide obtenues par reconstitution dans un milieux aqueux approprié de compositions d'immunoglobulines G polyclonales sous forme lyophilisée.

## Patentansprüche

1. Stabilisierende Formulierung für eine polyklonale Immunoglobulin-G-Zusammensetzung, **dadurch gekennzeichnet, dass** sie aus einem Zuckeralkohol, Glycin in einer Konzentration im Bereich zwischen 7 g/l und 10 g/l und einem anionischen Detergens in einer Konzentration im Bereich zwischen 20 und 50 ppm besteht, so dass sie für die Stabilisierung von polyklonalen Immunoglobulin-G-Zusammensetzungen in lyophilisierter Form geeignet ist.

2. Formulierung nach Anspruch 1, **dadurch gekennzeichnet, dass** der Zuckeralkohol Mannitol ist.

3. Formulierung nach Anspruch 2, **dadurch gekennzeichnet, dass** die Mannitolkonzentration im Bereich zwischen 30 g/l und 50 g/l liegt.

4. Polyklonale Immunoglobulin-G-Zusammensetzung in flüssiger Form, die als Stabilisator die stabilisierende Formulierung nach einem der Ansprüche 1 bis 3 umfasst.

5. Polyklonale Immunoglobulin-G-Zusammensetzung in lyophilisierter Form, die durch Lyophilisation einer polyklonalen Immunoglobulin-G-Zusammensetzung und der stabilisierenden Formulierung nach einem der Ansprüche 1 bis 3 erhalten wird.

6. Polyklonale Immunoglobulin-G-Zusammensetzung nach Anspruch 4, **dadurch gekennzeichnet, dass** sie nach einer Lagerung für 6 Monate bei Umgebungstemperatur einen Polymeranteil von weniger als 0,3 % aufweist.

7. Polyklonale Immunoglobulin-G-Zusammensetzung nach Anspruch 5, **dadurch gekennzeichnet, dass** sie nach einer Lagerung für 12 Monate bei Umgebungstemperatur oder für 6 Monate bei 40 °C einen Polymeranteil von weniger als 0,3 % aufweist.

8. Polyklonale Immunoglobulin-G-Zusammensetzung nach einem der Ansprüche 4 bis 7, **dadurch gekennzeichnet, dass** sie nach einer Lagerung während eines Zeitraums von 24 Monaten bei 4 °C einen Dimeranteil von weniger als 7 % aufweist.

9. Verwendung einer stabilisierenden Formulierung nach einem der Ansprüche 1 bis 3 als Stabilisator von polyklonalen Immunoglobulin-G-Zusammensetzungen in flüssiger Form, die durch Fraktionierung direkt aus Humanplasma erhalten wurden.

10. Verwendung einer stabilisierenden Formulierung nach einem der Ansprüche 1 bis 3 zur Stabilisierung von polyklonalen Immunoglobulin-G-Zusammensetzungen in lyophilisierter Form.

11. Verwendung einer stabilisierenden Formulierung nach einem der Ansprüche 1 bis 3 zur Stabilisierung von polyklonalen Immunoglobulin-G-Zusammensetzungen in flüssiger Form, die durch Rekonstitution in einen wässrigen Medium erhalten wurden, das für polyklonale Immunoglobulin-G-Zusammensetzungen in lyophilisierter Form geeignet ist.

## Claims

1. Stabilizing formulation for polyclonal immunoglobulins G compositions, **characterized in that** it includes a sugar alcohol, glycine in a concentration between 7 g/l and 10 g/l and a non-ionic detergent in a concentration between 20 and 50 ppm, in order to be suitable for the stabilisation of polyclonal immunoglobulins G compositions in liquid form and for the stabilisation of polyclonal immunoglobulins G compositions in lyophilised form.

2. Formulation according to claim 1, **characterized in that** the sugar alcohol is mannitol.

3. Formulation according to claim 2, **characterized in that** the concentration of mannitol is between 30 g/l and 50 g/l.

4. Polyclonal immunoglobulins G composition in liquid form comprising as a stabiliser, the stabilizing formulation according to any one of the claims 1 to 3.

5. Polyclonal immunoglobulins G composition in lyophilised form, obtained by lyophilisation of a polyclonal immunoglobulins G composition and the stabilising formulation according to any one of the claim 1 to 3.

6. Polyclonal immunoglobulins G composition according to claim 4, **characterized in that** it includes an amount of polymers less than 0.3% after a 6 months storage period at room temperature.

7. Polyclonal immunoglobulins G composition according to claim 5, **characterized in that** it includes an amount of polymers less than 0.3% after a 12 months storage period at room temperature or a 6 months storage period at 40°C.

8. Polyclonal immunoglobulins G composition according to any one of the claims 4 to 7, **characterized in that** it includes an amount of dimers less than 7% after a 24 months storage period at 4°C.

9. Use of a stabilising formulation according to any one of the claims 1 to 3, as a stabiliser for polyclonal immunoglobulin G compositions in liquid form obtained directly by fractioning of human plasma.

10. Use of a stabilising formulation according to any one of the claims 1 to 3, for the stabilisation of polyclonal immunoglobulin G composition in lyophilised form.

11. Use of a stabilising formulation according to any one of the claims 1 to 3, for the stabilisation of polyclonal immunoglobulin G composition in liquid form obtained after reconstitution in a suitable aqueous medium of an immunoglobulin G composition in lyophilised form.
